(19) **Europäisches Patentamt**

**European Patent Office**

**Office européen des brevets**

(11) **EP 0 626 011 B1**

(12) **EUROPEAN PATENT SPECIFICATION**

(45) Date of publication and mention
of the grant of the patent:
**28.11.2001  Bulletin 2001/48**

(51) Int Cl.[7]: **C12N 11/04**, C12M 1/40

(86) International application number:
**PCT/NO93/00017**

(21) Application number: **93903342.9**

(22) Date of filing: **20.01.1993**

(87) International publication number:
**WO 93/15190 (05.08.1993 Gazette 1993/19)**

(54) **A METHOD AND APPARATUS FOR CONTINUOUS PREPARATION OF BIOCATALYST PARTICLES**

VERFAHREN UND APPARAT ZUR KONTINUIERLICHEN HERSTELLUNG VON BIOKATALYTISCHEN PARTIKELN

PROCEDE ET APPAREIL DE PREPARATION EN CONTINU DE PARTICULES BIOCATALYTIQUES

(84) Designated Contracting States:
**AT BE CH DE DK FR GB IT LI NL SE**

(30) Priority: **22.01.1992  NO 920291**

(43) Date of publication of application:
**30.11.1994  Bulletin 1994/48**

(73) Proprietor: **NORSKE MEIERIER**
**N-0187 Oslo (NO)**

(72) Inventors:
 • **LYSBERG, Magne**
 **N-7078 Saupstad (NO)**
 • **STORR , Ivar**
 **N-7033 Trondheim (NO)**
 • **SVENDSEN, Hallvard**
 **N-7035 Trondheim (NO)**
 • **WINNBERG, Asgeir**
 **N-7075 Tiller (NO)**
 • **EIDE, Ola**
 **N-0667 Oslo (NO)**
 • **OTERHOLM, Bjarne**
 **N-1430 Äs (NO)**

(74) Representative: **Onn, Thorsten et al**
**Stockholms Patentbyra Zacco AB**
**Box 23101**
**104 35 Stockholm (SE)**

(56) References cited:
**EP-A- 0 211 626**          **EP-A- 0 216 182**
**DE-C- 3 417 899**

**Description**

The invention relates to a method and apparatus for continuous preparation of biocatalyst particles, where the particles have uniform size and spherical shape.

The term biocatalyst in this connection refers to a biological system comprising microorganisms or cells, as well as parts thereof, e.g., enzymes. By biocatalyst particles is meant biocatalysts that are fixed (immobilized) by means of a macroscopic carrier, e.g., a hydrocolloid gel, enabling the system to carry out metabolic processes.

Industrial utilization of biocatalyst particles has been limited by the possibility of producing such particles. Various processes for producing these particles have been described previously.

The simplest method is described in German patent DE 2835875 and is based on dripping an alginate solution out of a tube having a small diameter. The disadvantage of this method is that the size of the alginate beads (due to the high viscosity of the solution) is almost independent of the tube dimension and therefore cannot be varied in a simple way.

A further development of the aforementioned procedure is to blow out the droplet with pressurized air. It is thereby possible to vary the size of the droplets. The problem is the high production costs required for this method.

A third method is to subject the alginate solution to oscillation, thus causing the droplets to "shoot" out of a thin tube. This method has ample capacity, but produces varying sizes of alginate beads. Industrial evaluation of this method has found it to be too costly.

The fourth method is described in DE 3417899 C2 "Verfaren zur Herstellung perlförminger Biokatalysatoren und zur Steigerung der Durchsatzkapazität und Vorrictung zu einer Durchfürung." Said patent describes a production method for spherical immobilized biocatalysts. In this method alginate droplets are hurled out of a perforated cylinder and caught by a rotating centrifuge basin filled with $CaCl_2$-solution. Due to the rotation, the $CaCl_2$ solution climbs up along the wall of the centrifige basin. The rotating, perforated cylinder is placed down in the centrifuge basin so that the alginate beads that are hurled out migrate horizontally until they hit the $CaCl_2$ solution. The method has a high capacity and the size of the alginate beeds may be varied. The disadvantage of this method is that it is mechanically complicated and difficult to use in aseptic processes. By aseptic process, in this connection, is meant a process that does not contain any bacteria other than the desired ones. The alginate beads produced will end up in the rotating centifuge basin, and from there must be transferred continuously, in some way or other, to a production reactor.

EP 216 182 discloses a process for producing gel beads consisting of a colloidal suspension of microbid cells or enzyme in a colloidal solution. The particles are formed into droplets with different size and shape by a rotor which is rotating at varied speeds.

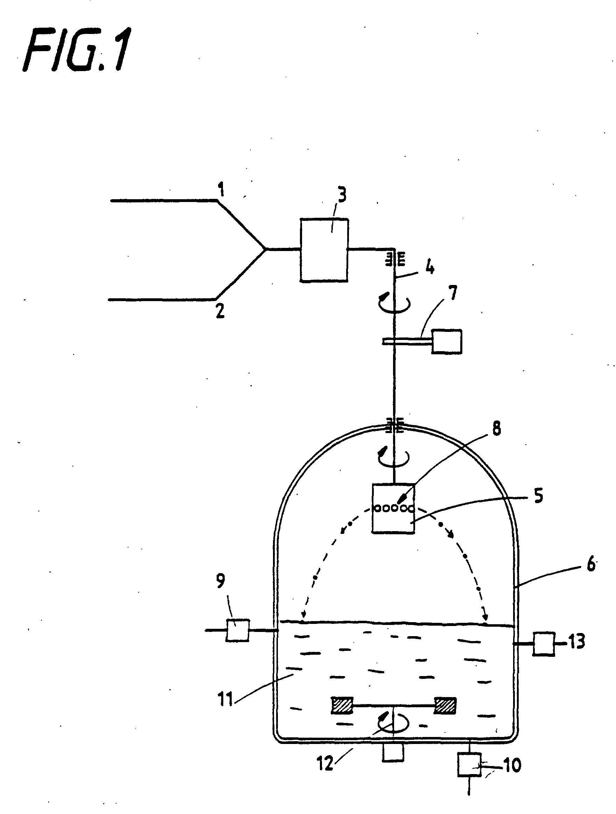

The invention's contribution is to move the perforated. rotating cylinder into the actual production reactor, which may also be an open and non-aseptic vessel. The method is characterized in that said cylinder (5) is placed in a reactor (6), and approximately monodisperse and approximately spherical particles are produced by rotation of the cylinder (5) at a velocity which hurls the beads directly down into the reactor liquid (11), and that the size of the beads is a function of the cylinder's velocity of rotation, which normally lies between 2.5 and 6300 $m/s^2$, preferably between 2.5 and 360 $m/s^2$. The hydrocolloid droplets that are hurled out are now picked up by the surface of the reactor, as shown on Figure 1. The need for complicated mechanical equipment such as a centrifuge is thus avoided. By producing the alginate beads in the production reactor, one also avoids the complicated step of transferring the hydrocolloid beads from the centrifuge to the reactor, and the system may be used for aseptic processes. With the process according to the invention, spherical particles of uniform size are produced, and the beads are metabolically active from the instant they hit the liquid surface in the reactor. It is not necessary for the beads to be placed in special liquids/mixtures for a period of time in order to mature. Nor is it necessary to separate them from the $CaCl_2$ liquid as described in the German patent DE 3417899.

The size of the particle beads that are hurled out may be regulated by means of the velocity of the rotating cylinder. With the method described in the invention, we obtain particles having approximately monodisperse spherical shape and a size deviation of up to 10%.

The invention will be further explained below, with reference to Figure 1 showing the rotary device for dispersing the beads (hereafter referred to as the droplet or bead hurler) placed in a potential reactor having supply lines for a gelling composition and biocatalyst, and a mixing chamber.

It is an advantage in the process according to the invention that the biocatalyst (1), e.g., bacteria, be rapidly transferred from a culturing apparatus (not shown) over to the mixing chamber (3) in order to avoid reducing the production potential. In mixing chamber (3) a solution of hydrocolloid, e.g., alginate (2), is mixed with the biocatalyst (1), to arrive at the desired concentration for the components. Then the mixture is conducted via a rigid tube (4) to the rotating, perforated cylinder (5) placed in the reactor (6). This rigid tube (4) is connected to a device (7) for regulating the velocity of rotation of tube (4) and cylinder (5). The reactor may also be equipped with a feed pipe (9) for supply of substrate and chemicals, a discharge means (10) for tapping biocatalyst particles, a stirring device (12), and an outlet (13) for removal of the product.

The droplet hurler (5) comprises a hollow cylinder sealed at one end thereof. The diameter of the cylinder may vary according to desired capacity. The upper end of the cylinder is connected at the center thereof to a rigid, hollow tube (4). This tube has two functions. When the tube rotates, the cylinder (5) also rotates with the same velocity and the hydrocolloid solution can be sent through the tube and down to the cylinder. The rotating cylinder has holes (8) drilled through the cylinder wall, enabling the biocatalyst solution in the hollow cylinder to be hurled out. Holes (8) may be distributed around the entire cylinder wall, and the diameter, placement and number of holes may vary depending on the desired capacity.

The landing radius for the droplets, which determines the minimum radius for the reactor, can be computed from the folloving formula:

$$r = v \cdot \sqrt{\frac{2h}{g}}$$

where

r:  radius
v:  velocity of the droplets as they leave the hurler
h:  height cf the holes above the liquid level
g:  acceleration of gravity

The formula's validity is shown in Example 1.

An important feature of the invention is that the size of the alginate bead is related to the velocity of rotation of the droplet hurler. This relationship can be determined by beginning with a droplet that is dripped out of a tube. The volume of such a droplet is expressed by the following equation:

$$V = \frac{2\pi \cdot r \cdot p}{\o \cdot d \cdot g}$$

where

V =  volume of the droplet
r =  diameter of the tube
p =  surface tension of the liquid
d =  density of the liquid
g =  acceleration of gravity
ø =  correction factor depending on the shape of the tube end

The variation in bead diameter for the gelled alginate droplets as a function of the velocity of rotation can be found by converting the equation. The droplet's volume is given by $4\pi R^3/3$, where R is the radius of the alginate droplet. Acceleration, in our case, is proportional to the velocity of rotation in the second power. The other factors entered in the relation can be combined into one simple correlation factor: A.

$$R = A \cdot \sqrt[3]{\frac{1}{(rpm)^2}}$$

where

R =  the diameter of the gel bead
A =  correlation factor
rpm =  revolutions per min.

The validity of the formula is shown in Example 2.

The described bead hurler has a high capacity, rendering it suitable for industrial use. The capacity in Example 1 and Example 2 is measured at 0.200 - 0.255 1 alginate solution per hour and hole at velocity of revolution of 500 to 1000 rpm. For the capacity measurement a bead hurler as described in Example 1 was used. If the bead hurler is produced with 100 holes, capacity will lie at about 20 1 alginate solution per hour, and this is sufficient for most purposes.

The formation of alginate beads from alginate solution requires a water solution of calcium ions (5-200 mM) in the reactor. Since anaerobic and facultative anaerobic bacteria, as well as a number of enzymes such as proteases and lipases, produce acids such as, respectively, energy metabolism products and reaction products, calcium ions can be added to the reactor as a neutralizing agent (for example $Ca(OH)_2$), either alone or in combination with other basic compounds. The addition of soluble calcium salts is also possible. If, e.g., milk, whey or other milk products are used in the reactor, the milk or milk products themselves will contain calcium ions, making it necessary only to add small extra amounts of calcium salts.

The reactor in the process may be of any type whatsoever that may be used with immobilized biocatalyst particles. The reactor thus may be, for example, a mixing tank reactor. A sugar solution, for example dairy effluent, whey, or ultrafiltered whey, is conducted continuously (9) into the reactor, and product solution is removed (13) at an approximately equal rate.

With a simple device (10) consisting of two computer-controlled ball valves and an intermediate volume, alginate beads may be tapped from the reactor so that the biocatalyst concentration in the reactor is held constant.

Example 1

Correlation between estimated landing radius and experimentally measured radius

The solution of Na-alginate (2% w/v Protanal LF 10/60) is hurled out of the rotating droplet hurler. The droplet hurler has an outer diameter of 4.6 cm and 24 holes having a diameter of 1.3 mm drilled into the inner cavity of the cylinder. This cavity is in contact with the hollow shaft on which the cylinder is mounted. Through the shaft the cylinder is supplied continuously with alginate solution.

TABLE 1

| Estimated and measured values for landing radius at varying velocities of rotation for the droplet hurler. | | |
|---|---|---|
| Velocity of rotation | Landing radius (cm) | |
| rpm | Theoretical | Experimental |
| 310 | 9.6 | 8.1 |
| 380 | 11.8 | 12.0 |
| 470 | 14.6 | 14.5 |
| 590 | 18.3 | 18.5 |
| 780 | 24.2 | 23.0 |
| 930 | 28.8 | 27.0 |
| 1150 | 35.7 | 30.5 |

Example 2

Estimated and measured values for alginate bead size as a function of velocity of rotation of the droplet hurler

The droplet hurler used is described in Example 1. The size 2of the alginate beads was measured with the aid of Dynatech Diffusion Zone Reader Mk II.

TABLE 2

| The size of the alginate bead as a function of the velocity of rotation of the droplet hurler. | | |
|---|---|---|
| rpm | Radius mm | Relative correlation factor |
| 1000 | 1.11 | 100 |
| 900 | 1.11 | 90.9 |
| 800 | 1.33 | 100 |
| 700 | 1.67 | 115 |
| 600 | 1.89 | 113 |
| 500 | 2.55 | 135 |

Example 3

Production of alginate immobilized lactic acid bacteria

The homofermenrative lactic acid bacterium Lactobacillus helveticus CNRZ 303 is stationarily cultivated in medium (0.5 1) at 40°C. After 20 hours the culture is innoculated in sanitary or, if desired, sterilized cultivation equipment. e.g., the membrane reactor shown in NO 174589.

A sugar solution in the form of ultrafilterec whey anc nitrogen source is conducted into the system. To the whey are added inorganic nutrients as shown in Table 3.

TABLE 3

| Inorganic nutrients added to ultrafiltered whey. | |
| --- | --- |
| $MgSO_4 \cdot 7H_{20}$ | 3 g |
| $MnSO_4 \cdot H_2O$ | 0.1 g |
| $K_2HPO_4$ | 4.4 g |
| $FeSO_4 . H_2O$ | 0.28 g |
| Whey | 1000 g |

Concentrated bacterium solution is pumped controllably out of the cultivation system when the bacterium concentration has reached the desired level, for example 60 g dry weight bacterium per liter. The bacterium solution is mixed with Na-alginate solution, for example, Protanal LF 10/60, to an alginate concentration of 0.5-4% (w/v). Preferred concentration is 1.5%. The bacterium concentration in the alginate beads is selected so as to allow the occurrence of only a minimal diffusion limitation during the production process. Typical values for the bacterium concentration are 1-100 g dry weight bacterium/kg alginate beads.

Lactic acid bacteria immobilized in alginate gel were produced as described in Example 1 and Example 2.

## Claims

1. Continuous process for production of biocatalyst particles where a biocatalyst (1) is mixed with hydrocolloid gelling agents, e.g., alginate (2) in a mixing chamber (3), the mixed mass is further conducted in a tube (4) to a rotating, perforated cylinder (5), **characterized in that** said cylinder (5) is placed in a reactor (6), which is not a rotating centrifuge basin, and that the cylinder (5) is rotated at a velocity causing the beads to be hurled directly down into the reactor liquid (11).

2. A method according to claim 1, **characterized in that** the velocity of rotation of the cylinder is between 2.5 and 6300 rpm, preferably between 2.5 and 360 rpm.

3. A method according to claims 1-2, **characterized in that** the holes in the rotating cylinder vary in number and placement.

4. A method according to claims 1-3, **characterized in that** the diameter of the holes in the cylinder is from 0.2 mm to 4.0 mm, preferably 0.5-2.5 mm.

5. A method according to claims 1-4, **characterized in that** the biocatalyst particles produced have a radius of from 0.1 to 4.0 mm. preferably 1.0-2.5 mm. and the deviation may be up to 10%.

6. A method according to claims 1-5, **characterized in that** the process can take place under aseptic conditions.

7. A method according to claims 1-6, **characterized in that** there are used biocatalyst particles which do not contain gas bubbles.

8. A method according to claims 1-7, **characterized in that** the biocatalyst particles may contain from 1 to 100 g biocatalyst per kg biocatalyst particle.

**Patentansprüche**

1. Kontinuierliches Verfahren zur Herstellung von Biokatalysatorteilchen, wobei ein Biokatalysator (1) mit hydrokolloidalen Geliermitteln, z. B. Alginat, (2) in einer Mischkammer (3) gemischt wird, die gemischte Masse weiter in einem Rohr (4) zu einem rotierenden, perforierten Zylinder (5) geleitet wird, **dadurch gekennzeichnet, dass** der Zylinder (5) in einen Reaktor (6), welcher kein rotierendes Zentrifugengefäß ist, gestellt wird und dass der Zylinder (5) mit einer Geschwindigkeit rotiert wird, so dass die Kügelchen direkt nach unten in die Reaktorflüssigkeit (11) geschleudert werden.

2. Verfahren gemäß Anspruch 1, **dadurch gekennzeichnet, dass** die Geschwindigkeit der Rotation des Zylinders zwischen 2,5 und 6300 U/min, vorzugsweise zwischen 2,5 und 360 U/min, liegt.

3. Verfahren gemäß Anspruch 1 - 2, **dadurch gekennzeichnet, dass** die Löcher in dem rotierenden Zylinder in der Anzahl und Anordnung variieren.

4. Verfahren gemäß Anspruch 1 - 3, **dadurch gekennzeichnet, dass** der Durchmesser der Löcher in dem Zylinder zwischen 0,2 mm und 4,0 mm, vorzugsweise zwischen 0,5 - 2,5 mm liegt.

5. Verfahren gemäß Anspruch 1 - 4, **dadurch gekennzeichnet, dass** die erzeugten Biokatalysatorteilchen einen Radius von 0,1 bis 4,0 mm, vorzugsweise von 1,0 - 2,5 mm, aufweisen und die Abweichung bei bis zu 10 % liegen kann.

6. Verfahren gemäß Anspruch 1 - 5, **dadurch gekennzeichnet, dass** das Verfahren unter aseptischen Bedingungen stattfinden kann.

7. Verfahren gemäß Anspruch 1 - 6, **dadurch gekennzeichnet, dass** Biokatalysatorteilchen verwendet werden, welche keine Gasbläschen enthalten.

8. Verfahren gemäß Anspruch 1 - 7, **dadurch gekennzeichnet, dass** die Biokatalysatorteilchen 1 bis 100 g Biokatalysator pro kg Biokatalysatorteilchen enthalten können.


**Revendications**

1. Procédé continu de production de particules biocatalytiques où un biocatalyseur (1) est mélangé avec des agents de formation de gel hydrocolloïdal, par exemple de l'alginate (2) dans une chambre de mélange (3), la masse mélangée est en outre conduite dans un tube (4) vers un cylindre perforé (5), **caractérisé en ce que** ledit cylindre (5) est placé dans un réacteur (6), qui n'est pas un bassin centrifuge en rotation, et **en ce que** le cylindre (5) est entraîné en rotation à une vitesse qui amène les billes à être projetées directement dans le liquide de réacteur (11).

2. Procédé selon la revendication 1, **caractérisé en ce que** la vitesse de rotation du cylindre est entre 2,5 et 6300 tours par minute, de préférence entre 2,5 et 360 tours par minute.

3. Procédé selon les revendications 1 à 2, **caractérisé en ce que** les trous dans le cylindre rotatif varient en nombre et en emplacement.

4. Procédé selon les revendications 1 à 3, **caractérisé en ce que** le diamètre des trous dans le cylindre est de 0,2 à 4,0 mm, de préférence de 0,5 à 2,5 mm.

5. Procédé selon les revendications 1 à 4, **caractérisé en ce que** les particules biocatalytiques produites ont un rayon de 0,1 mm à 4,0 mm, de préférence 1,0 à 2,5 mm, et l'écart peut être jusqu'à 10%.

6. Procédé selon les revendications 1 à 5, **caractérisé en ce que** le procédé peut être mis en oeuvre dans des conditions aseptiques.

7. Procédé selon les revendications 1 à 6, **caractérisé en ce que** l'on utilise des particules biocatalytiques qui ne contiennent pas de bulles de gaz.

8. Procédé selon les revendications 1 à 7, **caractérisé en ce que** les particules biocatalytiques peuvent contenir de 1à 100 g de biocatalyseur par kg de particules biocatalytiques.

# FIG.1